# EUROPEAN PATENT SPECIFICATION

(11) **EP 1 287 017 B1**
(45) Date of publication and mention of the grant of the patent: **03.08.2005**
(21) Application number: 01928120.3
(22) Date of filing: 11.05.2001
(51) Int. Cl.: C07J 41/00, A61K 31/57, C07J 9/00, C07J 13/00, C07J 51/00, A61K 31/575, A61P 5/30, A61P 35/00

(54) **2-SUBSTITUTED PREGNA-1,3,5(10)-TRIENE AND CHOLA-1,3,5(10)-TRIENE DERIVATIVES AND THEIR BIOLOGICAL ACTIVITY**
2-SUBSTITUIRTE PREGNA-1,3,5(10)-TRIEN- UND CHOLA-1,3,5(10)-TRIEN- DERIVATE UND IHRE BIOLOGISCHE AKTIVITÄT
DERIVES PREGNA-1, 3, 5 (10) -TRIENE ET CHOLA-1, 3, 5 (10)-TRIENE 2-SUBSTITUES ET LEUR ACTIVITE BIOLOGIQUE

(30) Priority: 11.05.2000 US 203462 P
(43) Date of publication of application: 05.03.2003
(73) Proprietor: RESEARCH INSTITUTE FOR MEDICINE AND CHEMISTRY INC., Cambridge, Massachusetts 02142 (US)
(72) Inventor: HESSE, Robert, Henry, Winchester, MA 01890 (US); SETTY, Sundara, Katugam, Srinivasasetty, Acton, MA 01720 (US); PECHET, Maurice, Murdoch, Cambridge, MA 02138 (US); GILE, Michael, Methuen, MA 01844 (US)
(74) Representative: Marsden, John Christopher
(86) International application number: PCT/GB2001/002103
(87) International publication number: WO 2001/085755

(56) References cited:
- WO-A-00/68246
- US-A- 3 562 260
- CUSHMAN M ET AL: "SYNTHESIS, ANTITUBULIN AND ANTIMITOTIC ACTIVITY, AND CYTOTOXICITY OF ANALOGS OF 2-METHOXYESTRADIOL, AN ENDOGENOUS MAMMALIAN METABOLITE OF ESTRADIOL THAT INHIBITS TUBULIN POLYMERIZATION BY BINDING TO THE COLCHICINE BINDING SITE" JOURNAL OF MEDICINAL CHEMISTRY,US,AMERICAN CHEMICAL SOCIETY. WASHINGTON, vol. 38, no. 12, 1995, pages 2041-2049, XP002055798 ISSN: 0022-2623
- DATABASE CHEMABS [Online] CHEMICAL ABSTRACTS SERVICE, COLUMBUS, OHIO, US; MORITA, YOSHIMI ET AL: "3-Methoxy-19-nor-20-hydroxymethylpregna-1 ,3,5(10)-triene" retrieved from STN Database accession no. 92:181486 XP002172562 & JP 54 117456 A (MITSUBISHI CHEMICAL INDUSTRIES CO., LTD., JAPAN) 12 September 1979 (1979-09-12) & DATABASE WPI Section Ch, Week 197943 Derwent Publications Ltd., London, GB; Class B01, AN 1979-77929B & JP 54 117456 A
- DATABASE CHEMABS [Online] CHEMICAL ABSTRACTS SERVICE, COLUMBUS, OHIO, US; MORITA, YOSHIMI ET AL: "3-Hydroxy-19-nor-20-hydroxymethylpregna-1 ,3,5(10)-triene" retrieved from STN Database accession no. 92:147043 XP002172563 & JP 54 112849 A (MITSUBISHI CHEMICAL INDUSTRIES CO., LTD., JAPAN) 4 September 1979 (1979-09-04) & PATENT ABSTRACTS OF JAPAN vol. 003, no. 133 (C-063), 7 November 1979 (1979-11-07) & JP 54 112849 A
- DATABASE CHEMABS [Online] CHEMICAL ABSTRACTS SERVICE, COLUMBUS, OHIO, US; MORITA, YOSHIMI ET AL: "3-Acyloxy-19-nor-20-(2-tetrahydropyranyl) -oxymethylpregna-1,3,5(10)- trienes" retrieved from STN Database accession no. 92:198643 XP002172564 & JP 54 117454 A (MITSUBISHI CHEMICAL INDUSTRIES CO., LTD., JAPAN) 12 September 1979 (1979-09-12) & DATABASE WPI Section Ch, Week 197943 Derwent Publications Ltd., London, GB; Class B01, AN 1979-77927B & JP 54 117454 A
- DATABASE CHEMABS [Online] CHEMICAL ABSTRACTS SERVICE, COLUMBUS, OHIO, US; MORITA, YOSHIMI ET AL: "3-Hydroxy-19-nor-20-acyloxymethylpregna-1 ,3,5(10)-trienes" retrieved from STN Database accession no. 92:215635 XP002172565 & JP 54 112850 A (MITSUBISHI CHEMICAL INDUSTRIES CO., LTD., JAPAN) 4 September 1979 (1979-09-04) & PATENT ABSTRACTS OF JAPAN vol. 003, no. 133 (C-063), 7 November 1979 (1979-11-07) & JP 54 112850 A
- DATABASE CHEMABS [Online] CHEMICAL ABSTRACTS SERVICE, COLUMBUS, OHIO, US; MORITA, YOSHIMI ET AL: "3-Benzoyloxy-19-nor-20-hydroxymethylpregn a-1,3,5(10)-triene" retrieved from STN Database accession no. 92:215638 XP002172569 & JP 54 117455 A (MITSUBISHI CHEMICAL INDUSTRIES CO., LTD., JAPAN) 12 September 1979 (1979-09-12) -& DATABASE WPI Section Ch, Week 197943 Derwent Publications Ltd., London, GB; Class B01, AN 1979-77928B XP002172571 & JP 54 117455 A

## Description

This invention relates to novel sterol derivatives, more particularly to 2-substituted ring A aromatic sterol derivatives having a comparatively short 17-position hydrocarbyl side chain which terminates in an carboxaldehyde group or an optionally substituted hydroxyl group. Such compounds have been found to have cell modulating activity and may exhibit valuable antiproliferative and antiangiogenic effects.

WO-A-0068246 discloses a range of 3-sterols and O-protected derivatives having an aromatic A-ring and a 17-position side chain which terminates with an amine, amide or hydroxyl group attached to a tertiary carbon atom. These compounds exhibit potent effects on the modulation of cell growth and differentiation, for example as demonstrated by their ability to inhibit growth of cancer cells in vitro and in vivo, while possessing an advantageous therapeutic ratio by virtue of their low levels of calcaemic activity, for example as determined by their effects on serum calcium and phosphorus levels in rats. In this respect the activity of the compounds resembles that of various vitamin D analogues despite the fact that they have an intact tetracyclic nucleus and lack both the seco steroid triene system of vitamin D analogues and the ability to mimic a conjugated conformational isomer thereof.

It is also known that 2-methoxyoestradiol, which like 2-methoxyoestrone is a natural metabolite of oestradiol, prevents proliferation and promotes the death of cancer cells *in vivo.* Studies have suggested that these effects are at least in part mediated by inhibition or misdirection of tubulin polymerisation in a manner similar to that exhibited by colchicine; thus the metabolite has been observed to bind to the colchicine binding site of tubulin.

As noted by Cushman *et al.* in *J. Med. Chem. **38(12)*****,** pp. 2041-2049 [1995], 2-methoxyoestradiol has also been found to inhibit angiogenesis (the creation of new blood vessels); this is potentially an extremely valuable property in cancer treatment, since angiogenesis is required for the growth of solid tumours. Both Cushman *et al*. (*op. cit*.) and Lovely *et al*. in *J. Med. Chem*. ***39(9)*****,** pp. 1917-1923 [1996] report the synthesis and evaluation for cytotoxicity of various 2-methoxyoestradiol analogues; all the compounds investigated were 17-ones or optionally substituted 17-ols.

The antiproliferative and antiangiogenic effects of 2-methoxyoestradiol are discussed by Pribluda *et al*. in *Cancer and Metastatic Reviews **19(1-2),*** pp. 173-179 [2000], where it is stated that it targets both tumour cell and endothelial cell compartments by inducing apoptosis in rapidly proliferating cells and inhibiting blood vessel formation at several stages in the angiogenic cascade. It is also said to inhibit metastatic spread in several models.

Dubey *et al*. in *Biochem. Biophys. Comm. **278(1)*****,** pp. 27-33 [2000] report that endogenous methoxyoestradiols mediate the antimitogenic effects of oestradiol on vascular smooth muscle cells via oestrogen receptor-independent mechanisms.

WO-A-9933859 discloses a variety of 1,3,5-oestratrienes having heteroatom-containing hydrocarbyl side chains at the 17-position. The compounds are said to be oestrogen antagonists and there is no suggestion that they may exhibit antiproliferative or antiangiogenic effects or colchicine-like interference with tubulin polymerisation.

WO-A-9933858 discloses 3-sulphamoyloxy-1,3,5-oestratrienes which contain relatively short hydrocarbyl side chains at the 17-position and which are said to act as inhibitors of steroidal sulphatase enzymes. Again there is no suggestion that they may exhibit antiproliferative or antiangiogenic effects or colchicine-like interference with tubulin polymerisation.

The present invention is based on the unexpected finding that a range of 2-substituted ring A aromatic sterol derivatives having a comparatively short 17-position hydrocarbyl side chain which terminates in a carboxaldehyde (-CHO) group or an optionally O- and/or C-substituted hydroxymethyl group exhibit potent cell modulating activity. Tissue culture assays show such compounds to exhibit dose response curves characteristic of colchicine, suggesting that their antiproliferative activity derives at least in part from colchicine-like interference with tubulin polymerisation. Compounds of the invention may therefore inhibit angiogenesis in analogous but more potent manner compared to compounds such as 2-methoxyoestradiol. The compounds also show reduced but still significant binding to oestrogen receptors, suggesting that they may have further applications analogous to those of other non-uterotrophic oestrogen response modulators.

According to one embodiment of the invention there are provided compounds of formula (I) 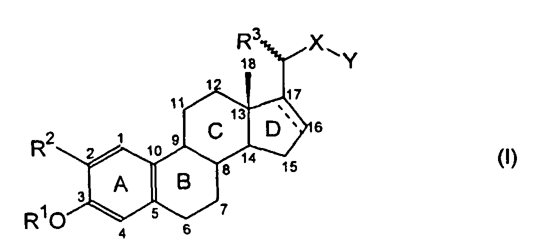 in which:
R¹ represents a hydrogen atom or an O-protecting group;
R² represents a hydroxyl, lower alkoxy, carboxaldehyde, lower alk-1-enyl or hydroxy- or lower alkoxy-substituted lower alkyl group;
R³ represents a methyl group having α- or β-configuration;
X represents a C₁₋₃ alkylene group or a valence bond;
Y represents a carboxaldehyde group or a group of formula -C(R⁴) (R⁵)OR¹ where R¹ is as defined above and R⁴ and R⁵, which may be the same or different, are each selected from hydrogen atoms, alkyl, alkenyl and alkynyl groups such that the total carbon content of R⁴ and R⁵ does not exceed three atoms, with the proviso that X is a valence bond when both R⁴ and R⁵ are other than hydrogen; and
the dotted line signifies that a double bond may optionally be present at the 16(17)-position.

O-protecting groups present as R¹ groups may, for example, comprise any suitable cleavable O-protecting group such as is known in the art. Representative groups include (i) etherifying groups such as silyl groups (e.g. tri(lower alkyl)silyl groups such as trimethylsilyl, triethylsilyl, triisopropylsilyl or t-butyldimethylsilyl; tri(aryl)silyl groups such as triphenylsilyl; and mixed alkyl-arylsilyl groups), lower (i.e. C₁₋₆) alkyl groups optionally interrupted by an oxygen atom (e.g. such as methyl, ethyl, methoxymethyl or methoxyethoxymethyl) or substituted by a lower (e.g. C₃₋₈) cycloalkyl group (e.g. as in cyclopentylmethyl) or by an acyloxy group (e.g. by a lower alkanoyloxy group, for example as in pivaloyloxymethyl), and cyclic ether groups (e.g. such as tetrahydropyranyl), and (ii) esterifying groups such as lower (e.g. C₁₋₆) alkanoyl (e.g. such as acetyl, propionyl, isobutyryl, pivaloyl or hemisuccinyl), aroyl (e.g. containing 7-15 carbon atoms, such as benzoyl or 4-phenylazobenzoyl), lower (e.g. C₁₋₆) alkane sulphonyl (e.g. such as methane sulphonyl or halogenated methane sulphonyl), arene sulphonyl (e.g. such as p-toluene sulphonyl), and sulphamoyl, for example groups of formula (R⁶) (R⁷)N.SO₂- where R⁶ and R⁷ are each independently selected from hydrogen atoms and lower (i.e. C₁₋₆) alkyl groups or together form a lower (e.g. C₃₋₁₀) alkylene chain optionally interrupted by one or more heteroatoms selected from O, N and S. It will be appreciated that R¹ at the 3-position may be the same as or different from any R¹ in the 17-position side chain.

Where R² represents a lower alkoxy group, this is a straight chain or branched C₁₋₆ alkoxy group such as a methoxy, ethoxy or propoxy group. Lower alk-1-enyl groups contain 2-6 carbon atoms, e.g. as in vinyl, prop-1-enyl and but-1-enyl groups. Representative hydroxy- and lower alkoxy-substituted lower alkyl groups include hydroxymethyl, 1-and 2-hydroxyethyl, 1-, 2- and 3-hydroxypropyl and corresponding methoxy- and ethoxy-substituted groups. Lower alkoxy-substituted lower alkyl groups contain up to 6 carbon atoms in total.

Where R³ in formula (I) is a methyl group in the α-configuration, the compounds have the 20R configuration characteristic of natural sterols such as cholesterol; where R³ is in the β-configuration the compounds have the 20S configuration of the corresponding epi-derivatives. It will be appreciated that the invention also embraces mixtures of the two isomers.

Where X is a (1-3 alkylene group this may, for example, be a methylene, ethylene or trimethylene group.

Where Y represents a group of formula -C(R⁴) (R⁵)OR¹ this may advantageously be an optionally O-protected hydroxymethyl group or a substituted hydroxymethyl group in which one of R⁴ and R⁵ is methyl, ethyl, vinyl, ethynyl or propargyl and the other is hydrogen, or in which R⁴ and R⁵ are both methyl.

Compounds of formula (I) in which R¹ is hydrogen, a metabolically labile O-protecting group (e.g. a lower alkanoyl group such as acetyl or hemisuccinyl; an acyloxymethyl group, for example a lower alkanoyloxymethyl group such as pivaloyloxymethyl; or a sulphonyl group, for example as in a sulphate or sulphamate group) or a C₁₋₆ alkyl etherifying O-protecting group such as methyl, ethyl or isobutyl may be useful directly in therapy. The use of compounds in which R¹ is a biolabile sulphamoyl group may be advantageous, since such groups will tend to inhibit steroid sulphatases which may otherwise degrade steroid-3-ols formed upon removal of such a protecting group from the 3-position. Compounds (I) containing non-metabolically labile O-protecting groups (e.g. bulky silyl ether groups such as triisopropyl, t-butyldimethylsilyl or triphenylsilyl) are principally of use as synthetic intermediates.

The cell modulating activity of active compounds according to the invention, combined with their substantial lack of adverse side effects, render them of interest both alone and as adjuncts in the management of diseases associated with abnormal cell proliferation, such as neoplastic disease, particularly myelogenous leukemias as well as neoplastic disease of the brain, breast, stomach, gastrointestinal tract, prostate, pancreas, uro-genital tract (male and female) and pulmonary neoplasia. Their ability to promote closure of mouse ear puches suggests their use, either alone or as adjuncts, as agents to promote wound healing.

Their cell modulating activity suggests that active compounds of the invention may, like other oestrogen response modulators, have additional utilities either alone or as adjuncts in the chemotherapy of infection and in other therapeutic modalities in which mononuclear phagocytes are involved, for example in treatment of bone disease (especially osteoporosis, osteopenia and osteodystrophy as in rickets or renal osteodystrophy), autoimmune disease, host-graft reaction, transplant rejection, inflammatory diseases (including modulation of immunoinflammatory reactions), neoplasias and hyperplasias, their potential utility in treatment of neoplasias and hyperplasias being evidenced by their ability to inhibit growth of a variety of human cancer cells. Additionally, they may be useful in treatment of dermatological diseases (for example including acne, alopecia, eczema, pruritus, psoriasis and skin aging, including photoaging), hypertension, rheumatoid arthritis, psoriatic arthritis, asthma, cognitive impairment and senile dementia (including Alzheimer's disease), in fertility control in both human and animal subjects, in lowering elevated serum cholesterol, and in management of disorders involving blood clotting (e.g. by dissolution of existing clots and/or by prevention of clotting). The invention embraces use of these compounds in the therapy or prophylaxis of such conditions and in the manufacture of medicaments for use in such treatment or prophylaxis.

Active compounds according to the invention may be formulated for administration by any convenient route, e.g. orally (including sublingually), parenterally, rectally or by inhalation; pharmaceutical compositions so formulated comprise a feature of the invention.

Orally administrable compositions may, if desired, contain one or more physiologically compatible carriers and/or excipients and may be solid or liquid. The compositions may take any convenient form including, for example, tablets, coated tablets, capsules, lozenges, aqueous or oily suspensions, solutions, emulsions, syrups, elixirs and dry products suitable for reconstitution with water or another suitable liquid vehicle before use. The compositions may advantageously be prepared in dosage unit form. Tablets and capsules according to the invention may, if desired, contain conventional ingredients such as binding agents, for example syrup, acacia, gelatin, sorbitol, tragacanth or polyvinyl-pyrollidone; fillers, for example lactose, sugar, maize-starch, calcium phosphate, sorbitol or glycine; lubricants, for example magnesium stearate, talc, polyethylene glycol or silica; disintegrants, for example potato starch; or acceptable wetting agents such as sodium lauryl sulphate. Tablets may be coated according to methods well known in the art.

Liquid compositions may contain conventional additives such as suspending agents, for example sorbitol syrup, methyl cellulose, glucose/sugar syrup, gelatin, hydroxymethylcellulose, carboxymethylcellulose, aluminium stearate gel or hydrogenated edible fats; emulsifying agents, for example lecithin, sorbitan monooleate or acacia; non-aqueous vehicles, which may include edible oils, for example vegetable oils such as arachis oil, almond oil, fractionated coconut oil, fish-liver oils, oily esters such as polysorbate 80, propylene glycol, or ethyl alcohol; and preservatives, for example methyl or propyl p-hydroxybenzoates or sorbic acid. Liquid compositions may conveniently be encapsulated in, for example, gelatin to give a product in dosage unit form.

Compositions for parenteral administration may be formulated using an injectable liquid carrier such as sterile pyrogen-free water, sterile peroxide-free ethyl oleate, dehydrated alcohol or propylene glycol or a dehydrated alcohol/propylene glycol mixture, and may be injected intravenously, intraperitoneally or intramuscularly.

Compositions for rectal administration may be formulated using a conventional suppository base such as cocoa butter or another glyceride.

Compositions for administration by inhalation are conveniently formulated for self-propelled delivery, e.g. in metered dose form, for example as a suspension in a propellant such as a halogenated hydrocarbon filled into an aerosol container provided with a metering dispense valve.

It may be advantageous to incorporate an antioxidant, for example ascorbic acid, butylated hydroxyanisole or hydroquinone in the compositions of the invention to enhance their storage life.

Where any of the above compositions are prepared in dosage unit form these may for example contain 2 µg - 100 mg of active compound according to the invention per unit dosage form; such dosage units may for example be administered 1-4 times per day. The compositions may if desired incorporate one or more further active ingredients.

A suitable daily dose of an active compound according to the invention may for example be in the range 2 µg - 400 mg per day, depending on factors such as the severity of the condition being treated and the age, weight and condition of the subject.

Compounds according to the invention may be prepared by any convenient method, for example by reaction of a compound containing a precursor for the desired 17-position side chain in one or more stages and with one or more reactants serving to form the desired 17-position side chain, followed if necessary and/or desired by removal of any O-protecting group(s).

Thus, for example, compounds of the invention may be prepared from appropriately 2-substituted, e.g. 2-hydroxylated or 2-alkoxylated, derivatives of oestrone by, for example, Wittig reaction with an ethylidene phosphorane to convert the 17-one to the corresponding 17(20)Z-ethylidene compound, following the procedure described by Krubiner and Oliveto, *J. Org. Chem*. ***31**,* pp. 24-26 [1965]. Alternatively, the corresponding E-isomer may be obtained following the procedure of Midland and Kwon, *Tetrahedron Lett. **23(20),*** pp. 2077-2080 [1982]. The thus-obtained alkenes may be subjected to conventional stereospecific hydroboration reactions followed by oxidative work-up with alkaline hydrogen peroxide solution (Krubiner, *op*. *cit*.) to afford the corresponding 20-ols, which may be oxidised to 20-ones with chromium trioxide (Krubiner, op. *cit.).* Wittig reaction with methoxymethylenetriphenyl-phosphorane followed by hydrolysis of the enol ether with aqueous acid gives a non-stereospecific compound (I) in which X is a valence bond and Y is an aldehyde group. Reduction with sodium borohydride gives a corresponding compound of formula (I) wherein X is a valence bond and Y is hydroxymethyl.

Compounds of the invention having a double bond at the 16(17)-position may, for example, be prepared stereospecifically by subjecting the appropriate E- or Z-17(20) ethylidene compound prepared as described above to a stereospecific ene reaction. For example, such ene reactions include treatment with formaldehyde, boron trifluoride and optionally acetic anhydride (Batcho et *al., Helv. Chim. Acta **64***, pp. 1682-1687 [1981]) to form compounds of formula (I) in which X is a valence bond and Y is hydroxymethyl or acetoxymethyl. In an alternative ene reaction, treatment with ethyl propiolate/diethyl aluminium chloride (Dauben and Brookhart, *J. Am. Chem. Soc. **103**,* pp. 237-238 [1980]) affords ethyl esters of Δ16,17 acids which may be reduced to give compounds in which Y is hydroxymethyl. Where appropriate the Δ16,17 compounds described above may be stereospecifically hydrogenated, e.g. catalytically, to form a single bond at the 16(17)-position.

The acetyl group in compounds in which Y is acetoxymethyl may be removed by hydrolysis and replaced by a leaving group such as tosyloxy. Homologation reactions may then be performed to increase the size of X, these including (i) treatment with a metal cyanide, hydrolysing the cyano group to yield a carboxy group or reducing the cyano group (e.g. with a metal hydride reducing agent such as diisobutyl aluminium hydride) to yield a carboxaldehyde group, and where appropriate reducing the carboxy or carboxaldehyde group (e.g. using sodium borohydride or lithium aluminium hydride) to yield a hydroxymethyl group which may in turn be subjected to tosylation and, if desired, further nucleophilic displacement; and (ii) treatment with a metallated derivative of an ester or thioester of acetic acid, with a derivative containing another carbanionic equivalent of acetic acid (e.g. a metallated derivative of acetonitrile), or with a metallated malonate ester (in which last instance the reaction product is partially hydrolysed to yield a monoester which may be decarboxylated by heating to yield a carboxylate ester), reducing the resulting ester or thioester product to an alcohol (e.g. using lithium aluminium hydride), and converting the resulting hydroxyl group to a leaving group, such as a tosylate group or a halogen atom, e.g. as hereinbefore described. Alternatively, compounds in which Y is a carboxaldehyde group may be homologated by Wittig reaction with methoxymethylenetriphenyl-phosphorane followed by hydrolysis of the resulting enol ether, e.g. with aqueous acid.

It will be appreciated that such procedures may be repeated as needed to yield compounds (I) in which X is a desired alkylene group.

Compounds where Y is a group -C(R⁴) (R⁵) OR¹ in which R⁴ and/or R⁵ are other than hydrogen may be prepared by conventional means, for instance from a corresponding aldehyde or ketone by reaction with an appropriate organometallic reagent, for example a Grignard reagent, metal acetylide, alkyl lithium or alkyl silane.

Compounds according to the invention may also be prepared from a 2-unsubstituted oestrogen by building up the desired 17-position side chain and then introducing the desired 2-substituent as a later step, for instance by following the procedures of Cushman *et al*. (*op*. cit.). The key 2-formyl compounds used in such procedures are conveniently prepared by formation of a 3-methoxymethyl ether, selective lithiation at the 2-position and formylation with dimethylformamide according to the procedure of Lovely *et al*. (*op. cit*.).

Sulphamoyl R¹ groups may be introduced by conventional methods such as reaction with an appropriate sulphamoyl chloride in the presence of a mild base, e.g. as described by Schwartz *et al*. in *Steroids **61***, pp. 710-717 [1996] or as described in WO-A-9933858. If, for example, it is desired selectively to introduce a sulphamoyl group at the 3-position, any hydroxyl group in the 17-position side chain may be protected during sulphamation, for example as a carboxylic ester (e.g. an acetate) or as a silyl ether; such protecting groups may subsequently be removed by hydrolysis without affecting the sulphamoyl group.

In general, O-protecting groups may, for example, be removed by conventional methods such as are well documented in the literature. Thus esterifying acyl groups may be removed by basic hydrolysis, e.g. using an alkali metal alkoxide in an alkanol. Etherifying groups such as silyl groups may be removed by acid hydrolysis or treatment with a fluoride salt, e.g. a tetraalkyl ammonium fluoride. The use of such acid-labile but base-stable protecting groups may be of particular advantage during homologation steps to build up a desired side chain, in view of the strongly basic conditions normally employed for such reactions.

The contents of all documents referred to in this specification are incorporated herein by reference.

The following non-limitative examples serve to illustrate the invention. All temperatures are in °C.

### Preparation 1

### 2-Methoxy-3-triisopropylsilyloxy-19-nor-pregn-1,3,5(10),17(20)Z-tetraene

Sodium hydride (294 mg, 50%) in dimethylsulphoxide (6 ml) was stirred at 70° for 1 hour, then cooled to room temperature. Ethyltriphenylphosphonium iodide (2.75 g) in dimethylsulphoxide (10 ml) was added dropwise and the mixture was stirred for 30 minutes. A solution of 2-methoxy-oestrone-3-triisopropylsilyl ether (600 mg, prepared by silylation of the 3-OH compound with triisopropylsilyl chloride and imidazole in dichloromethane overnight at room temperature) in dimethylsulphoxide (10 ml) was added dropwise. The resulting solution was stirred for 30 minutes, whereafter the temperature was raised to 70° and stirring was continued overnight. The reaction mixture was then cooled and worked up. Separation and purification of the products by chromatography gave the title compound (125 mg, see below) and the 3-OH analogue (300 mg): IR (CDCl₃) νₘₐₓ 1590, 3520 cm⁻¹; NMR (CDCl₃) δ 0.9 (s, 18-H's), 1.67 (d, =CH-CH's), 3.8 (s, OCH's), 4.7-5.2 (q, =CHMe), 6.5, 6.7 (s, 1,4-H's).

Silylation of this 3-OH compound (300 mg) as above and purification of the product by chromatography gave the title compound (370 mg): IR (CDCl₃) νₘₐₓ 1600 cm⁻¹; NMR (CDCl₃) δ 0.9 (s, 18-H's), 1.68 (d, =CH-CH's), 3.7 (s, OCH's), 4.7-5.3 (q, =CH-Me), 6.4, 6.6 (s, 1,4-H's).

### Example 1

### a) 2-Methoxy-3-triisopropylsilyloxy-19-nor-chol-1,3,5(10),16-tetraene-24-carboxylic acid methyl ester [Formula (I): R¹ = (i-Pr)₃Si, R² = CH₃O, R³ = α-CH₃, X = (CH₂)₂, Y = CO,OCH₃, Δ16 double bond]

Ethyl aluminium dichloride (1.4 ml, 2.4mmol, in toluene) was added dropwise to a solution of the product from Preparation 1 above (370 mg) in dichloromethane (4 ml) containing methyl acrylate (144 µl). The resulting mixture was stirred for 4 hours, whereafter further methyl acrylate (144 µl) was added and stirring was continued overnight. The reaction mixture was then worked up and the product was purified by chromatography to give the title compound (345 mg) : IR (CDCl₃) νₘₐₓ 1600, 1720 cm⁻¹; NMR (CDCl₃) δ 0.8 (s, 18-H's), 3.6 (s, OCH's), 5.1-5.4 (bs, 16-H's), 6.4, 6.58 (s, 1,4-H's).

### b) 2-Methoxy-3-triisopropylsilyloxy-19-nor-chol-1,3,5(10),16-tetraen-24-ol [Formula (I): R¹ = (i-Pr)₃Si, R² = CH₃O, R³ = α-CH₃, X = (CH₂)₂, Y = CH₂OH, Δ16 double bond]

Lithium aluminium hydride (1 ml of a 1M solution in ether) was added dropwise to a solution of the ester from (a) above (265 mg) in ether (5 ml), whereafter the reaction mixture was stirred for 30 minutes, diluted with ether and quenched with wet sodium sulphate, giving crude title compound (248 mg) : IR (CDCl₃) νₘₐₓ 1600, 3380-3660 cm⁻¹; NMR (CDCl₃) δ 0.8 (s, 18-H's), 3.3-3.8 (b, HOCH's), 3.7 (s, OCH's), 5.1-5.4 (bs, 16-H's), 6.4, 6.6 (s, 1,4-H's).

### c) 2-Methoxy-3-hydroxy-19-nor-chol-1,3,5(10),16-tetraen-24-ol [Formula (I): R¹ = H, R² = CH₃O, R³ = α-CH₃, X = (CH₂)₂, Y = CH₂OH, Δ16 double bond]

The title compound was prepared by desilylating the product of (b) above by treatment with tetrabutylammonium fluoride in tetrahydrofuran at room temperature overnight: IR (CDCl₃) νₘₐₓ 1590, 3200-3660 cm⁻¹; NMR (CDCl₃) δ 0.8 (s, 18-Me), 1.15 (d, 21-Me), 3.4-3.7 (t, 24-OH, OMe), 5.1-5.4 (ea m, 16-H), 6.5-6.63 (m, 1,4-H's).

### d) 2-Methoxy-3-triisopropylsilyloxy-19-nor-chol-1,3,5(10),16-tetraen-24-ol-24-sulphamate ester [Formula (I): R¹ = (iPr₃)Si, R² = CH₃O, R³ = α-CH₃, X = (CH₂)₃, Y = CH₂O.SO₂, NH₂]

Sulphamoyl chloride (60 mg) was added to a solution of the alcohol from (b) above (55 mg) and dimethylaminopyridine (62 mg) in methylene chloride (2 ml), and the resulting mixture was stirred for 2 hours. Work up and purification by preparative thin layer chromatography gave the title compound (60 mg): IR (CDCl₃) νₘₐₓ 1600, 3100-3600 cm⁻¹; NMR (CDCl₃) δ 0.8 (s, 18-Me), 3.7 (s, OMe), 3.9-4.4 (bt, 24-H's), 4.5-5.0 (b, NH's), 5.1- 5.5 (m, 16-H), 6.43, 6.63 (ea s, 1,4-H's).

### e) 2-Methoxy-3-hydroxy-19-nor-chol-1,3,5(10),16-tetraen-24-ol-24-sulphamate ester [Formula (I): R¹ = H, R² = CH₃O, R³ = α-CH₃, X = (CH₂)₃, Y = CH₂OSO₂HH₂]

The product from (d) above was desilylated as in (c) above to afford the title compound (39 mg): IR (CDCl₃) νₘₐₓ 1590, 3200-3600 cm⁻¹; NMR (CDCl₃) δ 0.8 (s, 18-Me), 1.08 (d, 21-H's), 3.8 (s, OMe), 3.9-4.4 (bt, 24-H's), 4.5-4.9 (b, NH's), 4.9-5.5 (m, 16-H), 6.53, 6.67 (ea s, 1,4-H's).

### Example 2

### a) 2-Methoxy-3-triisopropylsilyloxy-20α-acetoxymethyl-19-nor-pregna-1,3,5(10),16-tetraene [Formula (I): R¹ = (i-Pr)₃Si, R² = CH₃O, R³ = α-CH₃, X = valence bond, Y = CH₂OCOCH₃, Δ16 double bond]

A mixture of boron trifluoride etherate (6 µl) and acetic anhydride (0.66 ml) in dichloromethane (0.3 ml) was added dropwise to a solution of 2-methoxy-3-triisopropylsilyloxy-19-nor-pregn-1,3,5(10),17(20)Z-tetraene from Preparation 1 (0.20 g) in dichloromethane (1 ml) containing acetic anhydride (0.1 ml) and paraformaldehyde (13 mg). The mixture was stirred for 2 hours, whereafter saturated sodium hydrogen carbonate was added and stirring was continued for 3 hours. The product was isolated by extraction into dichloromethane and purified by chromatography to give the title compound (205 mg): IR (CDCl₃) νₘₐₓ 1605, 1725 cm⁻¹; NMR (CDCl₃) δ 0.73 (s, 18-H's), 1.97 (s, OCOCH's), 3.6 (s, OCH's), 3.7-4.3 (b, 22-H's), 5.2-5.5 (bs, 16-H's), 6.4, 6.57 (s, 1,4-H's).

### b) 2-Methoxy-3-triisopropylsilyloxy-20α-acetoxymethyl-19-nor-pregn-1,3,5(10)-triene [Formula (I): R¹ = (i-Pr)₃Si, R² = CH₃O, R³ = α-CH₃, X = valence bond, Y = CH₂OCOCH₃]

A solution of the product from (a) above (205 mg) in ethanol (5 ml) containing 5% platinum on carbon (40 mg) was stirred under hydrogen for 18 hours. Filtration and removal of the solvent afforded the title compound mixed with unreacted starting material (195 mg): IR (CDCl₃) νₘₐₓ 1600, 1720 cm⁻¹; NMR (CDCl₃) δ 0.73 (s, 18-H's [starting material]), 0.8 (s, 18-H's [product]), 2.0 (s, OCOCH's), 3.6 (s, OCH's), 3.4-4.3 (b, 22-H's), 5.1-5.5 (bs, 16-H's), 6.4, 6.6 (s, 1,4-H's).

### c) 2-Methoxy-3-triisopropylsilyloxy-20α-hydroxyethyl-19-nor-pregn-1,3,5(10)-triene [Formula (I): R¹ = (i-Pr)₃Si, R² = CH₃O, R³ = α-CH₃, X = valence bond, Y = CH₂OH] and 2-methoxy-3-triisopropylsilyloxy-20α-hydroxymethyl-19-nor-pregn-1,3,5(10),16-tetraene [Formula (I): R¹ = (i-Pr)₃Si, R² = CH₃O, R³ = α-CH₃, X = valence bond, Y = CH₂OH, Δ16 double bond]

Lithium aluminium hydride (0.5 ml of a 1M solution in ether) was added dropwise to the product mixture from (b) above (195 mg) in ether (4 ml). The resulting mixture was stirred for 30 minutes, treated with wet sodium sulphate and worked up to give a crude mixture of the title compounds (175 mg): IR (CDCl₃) νₘₐₓ 1600, 3620 cm⁻¹; NMR (CDCl₃) δ 0.73 (s, 18-H's [starting material]), 0.8 [s, 18-H's [products, ca. 1:1 mixture]), 3.7 (s, OCH's), 3.2-3.7 (b, 22-H's), 5.1-5.5 (bs, 16-H's), 6.4, 6.6 (s, 1,4-H's).

### d) 2-Methoxy-3-hydroxy-20α-hydroxymethyl-19-nor-pregn-1,3,5(10)-triene [Formula (I): R¹ = H, R² = CH₃O, R³ = α-CH₃, X = valence bond, Y = CH₂OH] and 2-methoxy-3-hydroxy-20α-hydroxymethyl-19-nor-pregn-1,3,5(10),16-tetraene [Formula (I): R¹ = H, R² = CH₃O, R³ = α-CH₃, X = valence bond, Y = CH₂OH, Δ16 double bond]

The product mixture from (c) above (45 mg) in tetrahydofuran (0.3 ml) was desilylated by treatment with tetrabuylammonium fluoride in tetrahydrofuran (0.3 ml) at room temperature overnight to give the title compound: (28 mg, semi-purified by preparative thin layer chromatrography): IR (CDCl₃) νₘₐₓ 1600, 3400-3660 cm⁻¹; NMR (CDCl₃) δ 0.73 (s, 18-H's [starting material]), 0.83 (s, 18-H's [products, ca. 1:1 mixture]), 3.8 (s, OCH's), 3.2-3.7 (b, 22-H's), 5.2-5.5 (bs, 16-H's), 6.4, 6.63 (s, 1,4-H's).

### Example 3

### 2-Methoxy-3-hydroxy-20α-hydroxymethyl-19-nor-pregn-1,3,5(10),16-tetraene [Formula (I): R¹ = H, R² = CH₃O, R³ = α-CH₃, X = valence bond, Y = CH₂OH, Δ16 double bond]

The title compound was prepared from the product of Example 2(a) by the procedures of Example 2(c) and (d): IR (CDCl₃) νₘₐₓ 1580, 3420-3660 cm⁻¹; NMR (CDCl₃) δ 0.83 (s, 18-Me), 1.07 (d, 21-Me), 3.3-4.0 (bm, 22-H's), 3.8 (s, OMe), 5.1-5.6 (bm, 16-H, OH), 6.47, 6.6 (2xd, 1,4-H's).

### Example 4

### 2-Methoxy-3-hydroxy-20α-acetoxymethyl-19-nor-pregn-1,3,5(10),16-tetraene [Formula (I): R¹ = H, R² = CH₃O, R³ = α-CH₃, X = valence bond, Y = CH₂O.CO.CH₃, Δ16 double bond]

The title compound was prepared by desilylating the product of Example 2(a) using the procedure of Example 2 (d) : IR (CDCl₃) νₘₐₓ 1580, 3420-3660 cm⁻¹; NMR (CDCl₃) δ 0.77 (s, 18-Me), 1.05 (d, 21-Me), 2.0 (s, COMe), 3.2-4.3 (bm, 22-H's), 3.8 (s, OMe), 5.0-5.6 (bm, 16-H, OH), 6.47, 6.6 (2xd, 1,4-H's).

### Example 5

### 2-Methoxy-3-hydroxy-20α-hydroxymethyl-19-nor-pregn-1,3,5(10)-triene [Formula (I): R¹ = H, R² = CH₃O, R³ = α-CH₃, X = valence bond, Y = CH₂OH]

A solution of the product from Example 3 (60 mg) in ethanol (6 ml) containing 5% platinum on charcoal (20 mg) was stirred under hydrogen overnight, whereafter the catalyst was removed by filtration, the solvent was evaporated and the thus-obtained crude product was purified by preparative thin layer chromatography to give the title compound (44 mg) : IR (CDCl₃) νₘₐₓ 1580, 3420-3640 cm⁻¹; NMR (CDCl₃) δ 0.73 (s, 18-Me), 1.07 (d, 21-Me), 3.3-3.7 (bm, 22-H's), 3.83 (s, OMe), 6.5, 6.63 (2xd, 1,4-H's).

### Example 6

### a) 2-Methoxy-3-hydroxy-20α-acetoxymethyl-19-nor-pregn-1,3,5(10),16-tetraene-3-O-sulphamate [Formula (I): R¹ = NH₂, SO₂, R² = CH₃O, R³ = α-CH₃, X = valence bond, Y = CH₂O.CO.CH₃, Δ16 double bond]

Sulphamoyl chloride (130 mg) was added to a solution of the product from Example 4 (84 mg) and di-tert.-butylaminopyridine (134 mg) in methylene chloride (5 ml), and the resulting mixture was stirred overnight. The mixture was then diluted with ether, washed with water then brine, and dried, whereafter the solvent was removed and the crude product was purified by preparative thin layer chromatography to give unreacted starting material (14 mg) and the title compound (61 mg): IR (CDCl₃) νₘₐₓ 1600, 1715, 3200-3500 cm⁻¹; NMR (CDCl₃) δ 0.8 (s, 18-Me), 2.0 (s, COMe), 3.6-4.3 (bm, 22-H's, OMe), 5.1-5.4 (bm, 16-H, OH), 6.77, 6.9 (2xd, 1,4-H's).

### b) 2-Methoxy-3-hydroxy-20α-acetoxymethyl-19-nor-pregn-1,3,5(10)-triene-3-O-sulphamate [Formula (I): R¹ = NH₂,SO₂, R² = CH₃O, R³ = α-CH₃, X = valence bond, Y = CH₂O, CO, CH₃]

The title compound is prepared by desilylating the product of Example 2(b) in accordance with the method of Example 2(d) and sulphamoylating the thus obtained 3-hydroxy compound as in (a) above. Alternatively the product of (a) above may be hydrogenated in accordance with the method of Example 2(b).

### Example 7

### a) 2-Methoxy-3-hydroxy-20α-hydroxymethyl-19-nor-pregn-1,3,5(10),16-tetraene-3-O-sulphamate [Formula (I): R¹ = NH₂.SO₂, R² = CH₃O, R³ = α-CH₃, X = valence bond, Y = CH₂OH, Δ16 double bond]

The product from Example 6(a) (40 mg) in methanol (3 ml) amd water (1 ml) containing sodium bicarbonate (38 mg) was stirred overnight. Work up and purification by preparative thin layer chromatography gave the title compound (16 mg): IR (CDCl₃) νₘₐₓ 3200-3600 cm⁻¹; NMR (CDCl₃) δ 0.83 (s, 18-Me), 3.3-3.83 (bm, 22-H's, OMe), 4.1-4.8 (bm), 5.2-5.6 (bm, 16-H, OH), 6.4, 7.1 (2xd, 1,4-H's).

### b) 2-Methoxy-3-hydroxy-20α-hydroxymethyl-19-nor-pregn-1,3,5(10)-triene-3-O-sulphamate [Formula (I): R¹ = NH₂.SO₂, R² = CH₃O, R³ = α-CH₃, X = valence bond, Y = CH₂OH]

The title compound is prepared by hydrolysing the product of Example 6(b) using the method of (a) above or by hydrogenating the product of (a) above using the method of Example 2(b).

### Example 8

### a) 3-Hydroxy-20α-hydroxymethyl-19-nor-pregn-1,3,5(10)-triene-3,22-bis-methoxymethyl ether [Formula (I): R¹ = CH₃OCH₂, R² = H, R³ = α-CH₃, X = valence bond, Y = CH₂OCH₂OCH₃]

The corresponding 3,22-diol (850 mg), which may be prepared by desilylating the product of Preparation 3(b) of WO-A-0068246, was dissolved in tetrahydrofuran (23 ml) containing diisopropylethylamine (3.9 ml) and was treated in the cold (0°) with methoxymethyl chloride (1 ml) added dropwise. The resulting mixture was allowed to warm to room temperature and was then refluxed overnight. Work up and purification by chromatography gave the title compound (900 mg) : IR (CDCl₃) νₘₐₓ 1600 cm⁻¹; NMR (CDCl₃) δ 0.7 (s, 18-Me), 1.08 (d, 21-Me), 3.27, 3.37 (ea s, OMe), 4.5, 5.0 (ea s, OCH₂O), 6.5-7.3 (m, 1,2,4-H's).

### b) 2-Formyl-3-hydroxy-20α-hydroxymethyl-19-nor-pregn-1,3,5(10)-triene-3,22-bis-methoxymethyl ether [Formula (I): R¹ = CH₃OCH₂, R² = H,CO, R³ = α-CH₃, X = valence bond, Y = CH₂OCH₂OCH₃]

A solution of s-butyl lithium in tetrahydrofuran (3.3ml, 4.3mM) was added dropwise at -78° to a solution of the ether from (a) above (440 mg) in tetrahydrofuran (6.6 ml). The resulting solution was stirred at -78° for 2 hours, treated with anhydrous dimethylformamide (2 ml) and then allowed to warm to room temperature overnight. Work up and purification by chromatography gave the title compound (323 mg): IR (CDCl₃) νₘₐₓ 1600, 1730 cm⁻¹; NMR (CDCl₃) δ 0.7 (s, 18-Me) , 1.08 (d, 21-Me), 3.3, 3.43 (ea s, OMe), 4.5, 5.13 (ea s, OCH₂O), 6.73, 7.57 (ea s, 1,4-H's), 10.2 (s, O=CH).

### c) 2,3-dihydroxy-20α-hydroxymethyl-19-nor-pregn-1,3,5(10)-triene-3,22-bis-methoxymethyl ether [Formula (I): R¹ = CH₃OCH₂, R² = HO, R³ = α-CH₃, X = valence bond, Y = CH₂OCH₂OCH₃]

A solution of m-chloroperbenzoic acid (204 mg) in methylene chloride (2 ml) was added dropwise to a solution of the formyl compound from (b) above (220 mg) in methylene chloride (4 ml) containing disodium hydrogen phosphate (245 mg). The mixture was stirred overnight at room temperature and was then worked up to give a 2-formate intermediate product. This intermediate was dissolved in methanol (4 ml) and the solution was deoxygenated with argon. Aqueous sodium hydroxide (1 ml, 1M) was added and the resulting solution was stirred for 2 hours and then brought to pH 7. Work up and purification by preparative thin layer chromatography gave the title compound (152 mg): IR (CDCl₃) νₘₐₓ 1590, 3200-3600 cm⁻¹; NMR (CDCl₃) δ 0.7 (s, 18-Me), 1.14 (d, 21-Me), 3.27, 3.42 (ea s, OMe), 4.5, 5.0 (ea s, OCH₂O), 5.5-5.7 (bm, OH), 6.5, 6.9 (ea s, 1,4-H's).

### d) 2-Ethoxy-3-hydroxy-20α-hydroxymethyl-19-nor-pregn-1,3,5(10)-triene-3,22-bis-methoxymethyl ether [Formula (I): R¹ = CH₃OCH₂, R² = C₂H₅O, R³ = α-CH₃, X = valence bond, Y = CH₂OCH₂OCH₃]

A solution of the 2-hydroxy compound from (c) above (90 mg) in anhydrous dimethylformamide (2.5 ml) containing anhydrous potassium carbonate (300 mg) was stirred for 10 minutes then treated with ethyl iodide (378 mg) followed by tetrabutylammonium iodide (4 mg). The resulting mixture was stirred for a further 5 hours, whereafter more tetrabutylammonium iodide (378 mg) was added and the mixture was stirred overnight. Work up and purification by preparative thin layer chromatography gave the title compound (72 mg): IR (CDCl₃) νₘₐₓ 1590 cm⁻¹; NMR (CDCl₃) δ 0.73 (s, 18-Me), 1.1 (d, 21-Me), 1.4 (t, Et), 3.35, 3.5 (ea s, OMe), 3.7-4.7 (q, Et), 4.58, 5.08 (ea s, OCH₂O), 5.5-5.7 (bm, OH), 6.75 (s, 1,4-H's).

### e) 2-Ethoxy-3-hydroxy-20α-hydroxymethyl-19-nor-pregn-1,3,5(1O)-triene [Formula (I): R¹ = H, R² = C₂H₅O, R³ = α-CH₃, X = valence bond, Y = CH₂OH]

The bis-ether from (d) above (72 mg) in tetrahydrofuran (1.6 ml) containing hydrochloric acid (0.8 ml, 6N) was stirred at room temperature for 2 days, after which time starting material was still observed to be present. Work up and purification by preparative thin layer chromatography gave unreacted starting material (25 mg) and the title compound (20 mg) : NMR (CDCl₃) δ 0.72 (s, 18-Me), 3.2-3.7 (bm, 22-H's), 3.7-4.3 (q, Et), 5.2-5.5 (bm, OH), 6.47, 6.61 (ea s, 1-,4-H's).

### f) 2-Formyl-3-hydroxy-20α-hydroxymethyl-19-nor-pregn-1,3,5(10)triene [Formula (I): R¹ = H, R² = H.CO, R³ = α-CH₃, X = valence bond, Y = CH₂OH]

A solution of the formyl compound from (b) above (100 mg) in tetrahydrofuran (2 ml) was treated with hydrochloric acid (6 ml, 6N) and the mixture was stored for 2 days at room temperature, after which time starting material was observed still to be present. Work up and purification by preparative thin layer chromatography gave the title compound (54 mg): IR (CDCl₃) νₘₐₓ 1600, 1650, 3100-3660 cm⁻¹; NMR (CDCl₃) δ 0.8 (s, 18-Me), 3.2-3.9 (m, 22-H's), 6.6-7.2 (ea s, 1,4-H's), 9.63 (s, O=CH).

### g) 2-Hydroxymethyl-3-hydroxy-20α-hydroxymethyl-19-nor-pregn-1,3,5(10)-triene [Formula (I): R¹ = H, R² = HOCH₂, R³ = α-CH₃, X = valence bond, Y = CH₂OH]

Lithium aluminium hydride (0.75 ml of a 1M solution in tetrahydrofuran) was added dropwise to a solution of the 2-formyl compound from (f) above (32 mg) in tetrahydrofuran (1.5 ml) at 0°. The resulting solution was allowed to warm to room temperature and was then stirred for 3 hours. Work up and purification by preparative thin layer chromatography gave the title compound (10 mg): IR (CDCl₃) νₘₐₓ 3500-3660 cm⁻¹; NMR (CDCl₃) δ 0.73 (s, 18-Me), 3.2-3.8 (m, 22-H's), 4.4-4.8 (bm, 2-CH₂OH), 6.6-7.2 (ea s, 1,4-H's).

### h) 2-Propenyl-3-hydroxy-20α-hydroxymethyl-19-norpregn-1,3,5(10)-triene [Formula (I): R¹ = H, R² = CH₃, CH=CH, R³ = α-CH₃, X = valence bond, Y = CH₂OH]

The title compound is obtained by adding a solution of the 2-formyl compound from (f) above in tetrahydrofuran to a solution of the ylide formed by reacting ethyltriphenylphosphonium bromide in tetrahydrofuran with lithium bis-trimethylsilylamide. Alternatively the protected 2-formyl compound from (b) above may be reacted with the same ylide, followed by removal of the methoxymethyl protecting groups as in (f) above.

### Example 9

### a) 2-Methoxy-3-triisopropylsilyloxy-20α-hydroxymethyl-19-nor-pregn-1,3,5,(10)-triene [Formula (I): R¹ = (i-Pr₃)Si, R² = CH₃O, R³ = α-CH₃, X = valence bond, Y = CH₂OH]

The 20α-acetoxymethyl product from Example 2(a) (360 mg) was first deacetylated by treatment with lithium aluminium hydride (1.2 ml, 2.5 eq.) in ether (7 ml) for 30 minutes at room temperature. The thus-obtained crude 20α-hydroxymethyl compound (200 mg) was then hydrogenated over 5% platinum on carbon (40 mg) in ethanol (10 ml). Filtration and removal of the solvent gave the title compound (175 mg) : IR (CDCl₃) νₘₐₓ 1600, 3300-3640 cm⁻¹; NMR (CDCl₃) δ 0.73 (s, 18-Me), 3.1-3.5 (m, 22-H; s, OMe), 6.43, 6.6(ea s, 1,4-H's).

### b) 2-Methoxy-3-triisopropylsilyloxy-19-nor-pregn-1,3,5,(10)-triene-20α-carboxaldehyde [Formula (I): R¹ = (i-Pr₃)Si, R² = CH₃O, R³ = α-CH₃, X = valence bond, Y = CHO]

The alcohol from (a) above (175 mg) in methylene chloride (4 ml) was treated with pyridinium dichromate (165 mg) for 2.5 hours. As the reaction was observed to be incomplete (50%, thin layer chromatography control) further pyridinium dichromate (165 mg) was added and the reaction mixture was stirred for a further 2.5 hours, by which time most of the starting material was observed to have been consumed. Work up and purification by preparative thin layer chromatography gave the title compound (125 mg): IR (CDCl₃) νₘₐₓ 1600, 1710 cm⁻¹; NMR (CDCl₃) δ 0.73 (s, 18-Me), 3.67 (s, OMe), 6.4, 6.6 (ea s, 1,4-H's), 9.38 (d, CH=O).

### c) 2-Methoxy-3-triisopropylsilyloxy-20α-(1-hydroxyethyl)-19-nor-pregn-1,3,5(10)-triene [Formula (I): R¹ = (i-Pr₃)Si, R² = CH₃O, R³ = α-CH₃, X = valence bond, Y = CH(CH₃)OH]

Methyl magnesium bromide (0.53 ml of a 1.4M solution in tetrahydrofuran/toluene) was added dropwise to a solution of the aldehyde from (b) above (125 mg) in ether at 0°. After 30 minutes at 0° and 30 minutes at room temperature the reaction was terminated by addition of saturated aqueous ammonium chloride and the product was worked up to give the title compound (125 mg): IR (CDCl₃) νₘₐₓ 1590, 3500-3640 cm⁻¹; NMR (CDCl₃) δ 0.7 (s, 18-Me), 3.3-3.67 (m, CHOH; s, OMe), 6.4, 6.6 (ea s, 1,4-H's).

### d) 2-Methoxy-3-hydroxy-20α-(1-hydroxyethyl)-19-nor-pregn-1,3,5(10)-triene [Formula (I): R¹ = H, R² = CH₃O, R³ = α-CH₃, X = valence bond, Y = CH(CH₃)OH]

The product from (c) above (45 mg) was desilylated by treatment with tetrabutylammonium fluoride (0.3 ml) in tetrahydrofuran (0.3 ml) (thin layer chromatography control, about 4 hours). Work up and purification by preparative thin layer chromatography gave the title compound (24 mg): IR (CDCl₃) νₘₐₓ 1580, 3460-3640 cm⁻¹; NMR (CDCl₃) δ 0.75 (s, 18-Me), 1.05 (d, 21-Me), 3.4-4.1 (m, CHOH; s, OMe), 6.5, 6.65 (ea s, 1,4-H's).

### e) 2-Methoxy-3-hydroxy-20α-(1-hydroxyprop-2-ynyl)-19-nor-pregn-1,3,5(10)-triene [Formula (I): R¹ = H, R² = CH₃O, R³ = α-CH₃, X = valence bond, Y = -CH(OH)C≡CH]

The title compound is prepared by treatment of the aldehyde from (b) above with sodium acetylide, followed by desilylation as in (d) above.

### f) 2-Methoxy-3-hydrogy-20α-(1-hydroxybut-3-ynyl)-19-nor-pregn-1,3,5(10)-triene [Formula (I): R¹ = H, R² = CH₃O, R³ = α-CH₃, X = valence bond, Y = CH(OH)CH₂C≡CH]

The title compound is prepared by treatment of the aldehyde from (b) above with propargyl aluminium reagent prepared as described in Example 3(b) of WO-A-0068246, followed by desilylation as in (d) above.

### g) 2-Methoxy-3-hydroxy-20α-(2-hydroxyprop-2-yl)-19-nor-pregn-1,3,5(10)-triene [Formula (I): R¹ = H, R² = CH₃O, R³ = α-CH₃, X = valence bond, Y = -C(OH) (CH₃)₂]

The title compound is obtained by oxidation of the 20α-(1-hydroxyethyl) compound from (c) above using pyridinium dichromate in accordance with the method of (b) above, followed by reaction with methyl magnesium bromide as in (c) above and desilylation as in (d) above.

### h) 2-Methoxy-3-triisopropylsilyloxy-19-nor-pregn-1,3,5(10)-triene-20β-carboxaldehyde [Formula (I) R¹ = (i-Pr₃)Si, R² = CH₃O, R³ = β-CH₃, X = valence bond, Y = CHO]

The title compound was obtained by isomerisation of the aldehyde from (b) above by treatment with 1,8-diazabicyclo[5.4.0]undec-7-ene in similar manner to that described in Preparation 5 of WO-A-9516672 and isolation of the newly formed isomer by chromatography (silica gel G, toluene/hexane, more polar isomer).

### i) 2-Methoxy-3-hydroxy-19-nor-pregn-1,3,5(10)-triene-20β-carboxaldehyde [Formula (I): R¹ = H, R² = CH₃O, R³ = β-CH₃, X = valence bond, Y = CHO]

The title compound is formed by desilylation of the epi-aldehyde from (h) above in accordance with the method of (d) above.

### j) 2-Methoxy-3-hydroxy-20β-hydroxymethyl-19-nor-pregn-1,3,5(10)-triene [Formula (I): R¹ = H, R² = CH₃O, R³ = β-CH₃, X = valence bond, Y = CH₂OH]

The title compound is obtained by reduction of the epi-aldehyde product from (h) above with sodium borohydride, followed by desilylation as in (d) above.

### k) 2-Methoxy-3-triisopropylsilyloxy-20α-(2-oxoethyl)-19-nor-pregn-1,3,5(10)-triene [Formula (I): R¹ = (i-Pr₃)Si, R² = CH₃O, R³ = α-CH₃, X = CH₂, Y = CHO]

The title compound is prepared from the aldehyde product of (b) above by reaction with methoxymethylenetriphenyl-phosphorane followed by acid hydrolysis of the intermediate enol ether, in similar manner to that described in Preparation 1 of WO-A-9945024.

### l) 2-Methoxy-3-hydroxy-20α-(2-oxoethyl)-19-nor-pregn-1,3,5(10)-triene [Formula (I): R¹ = H, R² = CH₃O, R³ = α-CH₃, X = CH₂, Y = CHO]

The title compound is prepared by desilylation of the product of (k) above as in (d) above.

### m) 2-Methoxy-3-hydroxy-20α-(2-hydroxyethyl)-19-nor-pregn-1,3,5(10)-triene [Formula (I): R¹ = H, R² = CH₃O, R³ = α-CH₃, X = CH₂, Y = CH₂OH]

The title compound is prepared by reduction of the aldehyde product of (k) above with sodium borohydride, followed by desilylation as in (d) above.

### Example 10

### 2-Methoxy-3-hydroxy-20α-hydroxymethyl-19-nor-pregn-1,3,5(10),16-tetraene-3,22-bis-O-sulphamate [Formula (I): R¹ = NH₂,SO₂, R² = CH₃O, R³ = α-CH₃, X = valence bond, Y = CH₂OSO₂NH₂, Δ16 double bond]

Sulphamoyl chloride (62 mg) was added to a solution of the product from Example 3 (16 mg) and dimethylaminopyridine (60 mg) in methylene chloride (1 ml), and the resulting mixture was stirred overnight. The mixture was then diluted with ether, washed with water then brine, and dried, whereafter the solvent was removed and the crude product was purified by preparative thin layer chromatography to give the title compound (10 mg): IR (CDCl₃) νₘₐₓ 1620, 3100-3600 cm⁻¹; NMR (CDCl₃) δ 0.8 (s, 18-Me), 1.12 (d, 21-Me), 3.77 (OMe), 5.0-5.5 (bm, 16-H), 6.73, 6.87 (ea s, 1,4-H's).

### Example 11

### a) 2-Methoxy-3-triisopropylsilyloxy-20α-hydroxymethyl-19-nor-pregn-1,3,5(10),16-tetraene-22-O-sulphamate [Formula (I): R¹ = (i-Pr₃)Si, R² = CH₃O, R³ = α-CH₃, X = valence bond, Y = CH₂OSO₂NH₂, Δ16 double bond]

Sulphamoyl chloride (60 mg) was added to a solution of 2-methoxy-3-triisopropylsilyloxy-20α-hydroxymethyl-19-nor-pregn-1,3,5(10),16-tetraene [Formula (I) : R¹ = (i-Pr₃)Si, R² = CH₃O, R³ = α-CH₃, X = valence bond, Y = CH₂OH, Δ16 double bond, obtained from the first step of Example 9(a)] (50 mg) and dimethylaminopyridine (60 mg) in methylene chloride (2.5 ml) and the resulting mixture was stirred for 1 hour. The mixture was then diluted with ether, washed with water then brine, and dried, whereafter the solvent was removed and the crude product was purified by preparative thin layer chromatography to give the title compound (55 mg): IR (CDCl₃) νₘₐₓ 1590, 3200-3500 cm⁻¹; NMR (CDCl₃) δ 0.83 (s, 18-Me), 3.7 (s, OMe), 4.4-4.8 (bm, NH's), 5.2-5.5 (bm, 16-H), 6.4, 6.6 (ea s, 1,4-H's).

### b) 2-Methoxy-3-hydroxy-20α-hydroxymethyl-19-nor-pregn-1,3,5(10),16-tetraene-22-O-sulphamate [Formula (I): R¹ = H, R² = CH₃O, R³ = α-CH₃, X = valence bond, Y = CH₂OSO₂NH₂, Δ16 double bond]

The silyl ether from (a) above (55 mg) was desilylated by treatment with tetrabutylammonium fluoride in tetrahydrofuran at room temperature for 3 hours and worked up and purified by preparative thin layer chromatography to give the title compound (30 mg): IR (CDCl₃) νₘₐₓ 1590, 3200-3600 cm⁻¹.

## Claims

1. Compounds of formula (I) 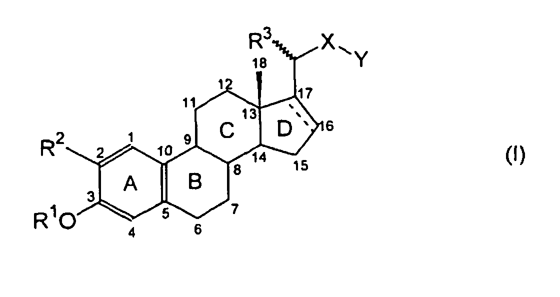 in which:
R¹ represents a hydrogen atom or an O-protecting group;
R² represents a hydroxyl, C₁₋₆ alkoxy, carboxaldehyde, C₂₋₆ alk-1-enyl or hydroxy- or alkoxy-substituted alkyl group containing up to 6 carbon atoms in total;
R³ represents a methyl group having α- or β-configuration;
X represents a C₁₋₃ alkylene group or a valence bond;
Y represents a carboxaldehyde group or a group of formula -C(R⁴) (R⁵)OR¹ where R¹ is as defined above and R⁴ and R⁵, which may be the same or different, are each selected from hydrogen atoms, alkyl, alkenyl and alkynyl groups such that the total carbon content of R⁴ and R⁵ does not exceed three atoms, with the proviso that X is a valence bond when both R⁴ and R⁵ are other than hydrogen; and
the dotted line signifies that a double bond may optionally be present at the 16(17)-position.

2. Compounds as claimed in claim 1 wherein R² represents a hydroxy or C₁₋₆ alkoxy group and Y is a carboxaldehyde group or a group of formula -C(R⁴) (R⁵)OR¹ where R¹ is as defined in claim 1 and R⁴ and R⁵ both represent hydrogen atoms.

3. Compounds as claimed in claim 1 wherein Y is a group of formula -C(R⁴) (R⁵)OR¹ in which R¹ is as defined in claim 1, one of R⁴ and R⁵ is methyl, ethyl, vinyl, ethynyl or propargyl and the other is hydrogen, or R⁴ and R⁵ both represent methyl groups.

4. Compounds as claimed in any of the preceding claims wherein each R¹ is selected from hydrogen atoms, C₁₋₆ alkyl groups and metabolically labile O-protecting groups.

5. Compounds as claimed in claim 4 wherein said metabolically labile O-protecting groups are sulphamoyl groups.

6. Compounds as claimed in any of the preceding claims wherein R² is selected from methoxy, ethoxy, propoxy, vinyl, prop-1-enyl, but-1-enyl, hydroxymethyl, hydroxyethyl, hydroxypropyl, methoxymethyl, methoxyethyl, methoxypropyl, ethoxymethyl, ethoxyethyl and ethoxypropyl groups.

7. The compounds according to claim 1:
2-methoxy-3-hydroxy-19-nor-chol-1,3,5(10),16-tetraen-24-ol;
2-methoxy-3-hydroxy-19-nor-chol-1,3,5(10),16-tetraen-24-ol-24-sulphamate ester;
2-methoxy-3-hydroxy-20α-hydroxymethyl-19-nor-pregn-1,3,5(10)-triene;
2-methoxy-3-hydroxy-20α-hydroxymethyl-19-nor-pregn-1,3,5(10),16-tetraene;
2-methoxy-3-hydroxy-20a-acetoxymethyl-19-nor-pregn-1,3,5(10),16-tetraene;
2-methoxy-3-hydroxy-20α-acetoxymethyl-19-nor-pregn-1,3,5(10),16-tetraene-3-O-sulphamate;
2-methoxy-3-hydroxy-20α-acetoxymethyl-19-nor-pregn-1,3,5(10)-triene-3-O-sulphamate;
2-methoxy-3-hydroxy-20α-hydroxymethyl-19-nor-pregn-1,3,5(10),16-tetraene-3-O-sulphamate;
2-methoxy-3-hydroxy-20α-hydroxymethyl-19-nor-pregn-1,3,5(10)-triene-3-O-sulphamate;
2-ethoxy-3-hydroxy-20α-hydroxymethyl-19-nor-pregn-1,3,5(10)-triene;
2-hydroxymethyl-3-hydroxy-20α-hydroxymethyl-19-nor-pregn-1,3,5(10)-triene;
2-propenyl-3-hydroxy-20α-hydroxymethyl-19-nor-pregn-1,3,5(10)-triene;
2-methoxy-3-hydroxy-20α-(1-hydroxyethyl)-19-nor-pregn-1,3,5(10)-triene;
2-methoxy-3-hydroxy-20α-(1-hydroxyprop-2-ynyl)-19-nor-pregn-1,3,5(10)-triene;
2-methoxy-3-hydroxy-20α-(1-hydroxybut-3-ynyl)-19-nor-pregn-1,3,5(10)-triene;
2-methoxy-3-hydroxy-20α-(2-hydroxyprop-2-yl)-19-nor-pregn-1,3,5(10)-triene;
2-methoxy-3-hydroxy-19-nor-pregn-1,3,5(10)-triene-20β-carboxaldehyde;
2-methoxy-3-hydroxy-20β-hydroxymethyl-19-nor-pregn-1,3,5(10)-triene;
2-methoxy-3-hydroxy-20α-(2-oxoethyl)-19-nor-pregn-1,3,5(10)-triene;
2-methoxy-3-hydroxy-20α-(2-hydroxyethyl)-19-nor-pregn-1,3,5(10)-triene;
2-methoxy-3-hydroxy-20α-hydroxymethyl-19-nor-pregn-1,3,5(10),16-tetraene-3,22-bis-O-sulphamate; and
2-methoxy-3-hydroxy-20α-hydroxymethyl-19-nor-pregn-1,3,5(10),16-tetraene-22-O-sulphamate.

8. Active compounds of formula (I) as claimed in any of the preceding claims for use in management of neoplastic disease; as agents to promote wound healing; in treatment of bone diseases, autoimmune disease, host-graft reaction, transplant rejection, inflammatory diseases, neoplasias or hyperplasias, dermatological diseases, hypertension, rheumatoid arthritis, psoriatic arthritis, asthma, cognitive impairment or senile dementia; in fertility control in human or animal subjects; in lowering elevated serum cholesterol or in management of disorders involving blood clotting.

9. Active compounds of formula (I) as claimed in any of claims 1 to 7 for use as antiangiogenics.

10. The use of an active compound of formula (I) as claimed in any of claims 1 to 7 for the manufacture of a medicament for use in management of neoplastic disease; as agents to promote wound healing; in treatment of bone diseases, autoimmune disease, host-graft reaction, transplant rejection, inflammatory diseases, neoplasias or hyperplasias, dermatological diseases, hypertension, rheumatoid arthritis, psoriatic arthritis, asthma, cognitive impairment or senile dementia; in fertility control in human or animal subjects; in lowering elevated serum cholesterol or in management of disorders involving blood clotting.

11. Pharmaceutical compositions comprising an active compound of formula (I) as claimed in any of claims 1 to 7 in admixture with one or more physiologically acceptable carriers or excipients.

## Patentansprüche

1. Verbindungen der Formel (I) 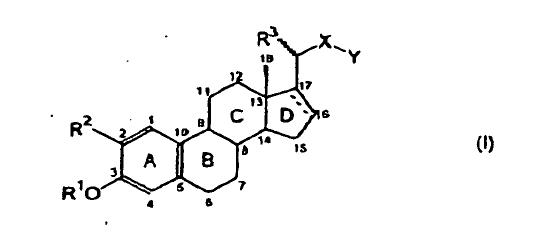 worin:
R¹ für ein Wasserstoffatom oder eine O-Schutzgruppe steht;
R² für eine Hydroxyl-, C₁₋₆-Alkoxy-, Carboxaldehyd-, C₂₋₆-Alk-1-enyl- oder Hydroxy- oder Alkoxy-substitulerte Alkylgruppe, die insgesamt bis zu 6 Kohlenstoffatome enthält, steht;
R³ für eine Methylgruppe mit α- oder β-Konfiguration steht;
X für eine C₁₋₃-Alkylengruppe oder eine Valenzbindung steht;
Y für eine Carboxaldehydgruppe oder eine Gruppe der Formel -C(R⁴)(R⁵)OR¹ steht, worin R¹ die oben angegebene Bedeutung hat und R⁴ und R⁵, die gleich oder verschieden sein können, jeweils ausgewählt sind unter Wasserstoffatomen, Alkyl-, Alkenyl- und Alkinyl-Gruppen, wobei der Gesamtkohlenstoffgehalt von R⁴ und R⁵ drei Atome nicht übersteigt, mit der Maßgabe, dass X eine Valenzbindung ist, falls sowohl R⁴ als auch R⁵ nicht Wasserstoff sind; und die gestrichelte Linie bedeutet, dass eine Doppelbindung gegebenenfalls in der 16(17)-Position vorliegen kann.

2. Verbindungen nach Anspruch 1, worin R² für eine Hydroxy- oder C₁₋₆-Alkoxygruppe steht und Y eine Carboxaldehydgruppe oder eine Gruppe der Formel -C(R⁴)(R⁵)OR¹ ist, worin R¹ die in Anspruch 1 angegebene Bedeutung hat und sowohl R⁴ als auch R⁵ für Wasserstoffatome stehen.

3. Verbindungen nach Anspruch 1, worin Y eine Gruppe der Formel C(R⁴)(R⁵)OR¹ ist, worin R¹ die in Anspruch 1 angegebene Bedeutung hat, einer der Reste R⁴ und R⁵ Methyl, Ethyl, Vinyl, Ethinyl oder Propargyl und der andere Wasserstoff ist, oder sowohl R⁴ als auch R⁶ für Methylgruppen stehen.

4. Verbindungen nach einem der vorhergehenden Ansprüche, worin R¹ jeweils unter Wasserstoffatomen, C₁₋₆-Alkylgruppen und metabolisch labilen O-Schutzgruppen ausgewählt ist.

5. Verbindungen nach Anspruch 4, worin die metabolisch labilen O-Schutzgruppen Sulphamoylgruppen sind.

6. Verbindungen nach einem der vorhergehenden Ansprüche, worin R² unter Methoxy-, Ethoxy-, Propoxy-, Vinyl-, Prop-1-enyl-, But-1-enyl-, Hydroxymethyl-, Hydroxyethyl-, Hydroxypropyl-, Methoxymethyl-, Methoxyethyl-, Methoxypropyl-, Ethoxymethyl-, Ethoxyethyl- und Ethoxypropyl-Gruppen ausgewählt ist.

7. Verbindungen nach Anspruch 1:
2-Methoxy-3-hydroxy-19-nor-chol-1,3,5(10),16-tetraen-24-ol;
2-Methoxy-3-hydroxy-19-nor-chol-1,3,5(10),16-tetraen-24-ol-24-sulphamatester;
2-Methoxy-3-hydroxy-20α-hydroxymethyl-19-nor-pregn-1,3,5(10)-trien;
2-Methoxy-3-hydroxy-20α-hydroxymethyl-19-nor-preg-1,3,5(10),16-tetraen;
2-Methoxy-3-hydroxy-20α-acetoxymethyl-19-nor-pregn-1,3,5(10),16-tetraen;
2-Methoxy-3-hydroxy-20α-acetoxymethyl-19-nor-pregn-1,3,5(10),16-tetraen-3-O-sulphamat;
2-Methoxy-3-hydroxy-20α-acetoxymethyl-19-nor-pregn-1,3,5(10)-trien-3-O-sulphamat;
2-Methoxy-3-hydroxy-20α-hydroxymethyl-19-nor-pregn-1,3,5(10),16-tetraen-3-O-sulphamat;
2-Methoxy-3-hydroxy-20α-hydroxymethyl-19-nor-pregn-1,3,5(10)-trien-3-O-sulphamat;
2-Ethoxy-3-hydroxy-20α-hydroxymethyl-19-nor-pregn-1,3,5(10)-trien;
2-Hydroxymethyl-3-hydroxy-20α-hydroxymethyl-19-nor-pregn-1,3,5(10)-trien;
2-Propenyl-3-hydroxy-20α-hydroxymethyl-19-nor-pregn-1,3,5(10)-trien;
2-Methoxy-3-hydroxy-20α-(1-hydroxyethyl)-19-nor-pregn-1,3,5(10)-trien;
2-Methoxy-3-hydroxy-20α-(1-hydroxyprop-2-inyl)-19-nor-pregn-1,3,5(10)-trien;
2-Methoxy-3-hydroxy-20α-(1-hydroxybut-3-inyl)-19-nor-pregn-1,3,5(10)-trien;
2-Methoxy-3-hydroxy-20α-(2-hydroxyprop-2-yl)-19-nor-pregn-1,3,5(10)-trien;
2-Methoxy-3-hydroxy-19-nor-pregn-1,3,5(10)-trien-20β-carboxaldehyd;
2-Methoxy-3-hydroxy-20β-hydroxymethyl-19-nor-pregn-1,3,5(10)-trien;
2-Methoxy-3-hydroxy-20α-(2-oxoethyl)-19-nor-pregn-1,3,5(10)-trien;
2-Methoxy-3-hydroxy-20α-(2-hydroxyethyl)-19-nor-pregn-1,3,5(10)-trien;
2-Methoxy-3-hydroxy-20α-hydroxymethyl-19-nor-pregn-1,3,5(10),16-tetraen-3,22-bis-O-sulphamat; und
2-Methoxy-3-hydroxy-20α-hydroxymethyl-19-nor-pregn-1,3,5(10),16-tetraen-22-O-sulphamat.

8. Aktive Verbindungen der Formel (I) nach einem der vorhergehenden Ansprüche zur Behandlung neoplastischer Erkrankung; als Mittel zur Förderung der Wundheilung; bei der Behandlung von Knochenerkrankungen, Autoimmunerkrankung. Wirt-Transplantat-Reaktion, Transplantatabstoßung, entzündlichen Erkrankungen, Neoplasien oder Hyperplasien, dermatologischen Erkrankungen, Hypertonie, rheumatoider Arthritis, psoriatischer Arthritis, Asthma, kognitiven Störungen oder seniler Demenz: bei der Steuerung der Fruchtbarkeit bei Mensch oder Tier; bei der Absenkung erhöhten Serumcholesterins oder der Behandlung von Störungen, an denen eine Blutgerinnung beteiligt ist.

9. Aktive Verbindungen nach Formel (I) nach einem der Ansprüche 1 bis 7 zur Verwendung als Antiangiogenetika.

10. Verwendung einer aktiven Verbindung nach Formel (I) nach einem der Ansprüche 1 bis 7 zur Herstellung eines Arzneimittels zur Behandlung neoplastischer Erkrankung; als Mittel zur Förderung der Wundheilung; bei der Behandlung von Knochenerkrankungen, Autoimmunerkrankung, Wirt-Transplantat-Reaktion, Transplantatabstoßung, entzündlichen Erkrankungen, Neoplasien oder Hyperplasien, dermatologischen Erkrankungen, Hypertonie, rheumatoider Arthritis, psoriatischer Arthritis, Asthma, kognitiven Störungen oder seniler Demenz; bei der Steuerung der Fruchtbarkeit bei Mensch oder Tier; bei der Absenkung erhöhten Serumcholesterins oder der Behandlung von Störungen, an denen eine Blutgerinnung beteiligt ist

11. Pharmazeutisches Mittel umfassend eine aktive Verbindung nach Formel (I) nach einem der Ansprüche 1 bis 7 im Gemisch mit einem oder mehreren physiologisch verträglichen Trägem oder Exzipienten.

## Revendications

1. Composés de formule (I) 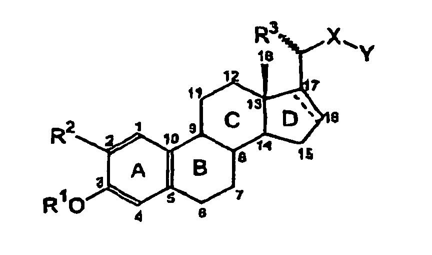 dans laquelle
R¹ représente un atome d'hydrogène ou un groupe protecteur de O ;
R² représente un groupe hydroxyle, alcoxy en C₁₋₆, carboxaldéhyde, alc-1-ényle en C₂₋₆ ou alkyle substitué par un groupe hydroxy ou alcoxy contenant jusqu'à 6 atomes de carbone au total ;
R³ représente un groupe méthyle ayant une configuration α ou β ;
X représente un groupe alkylène en C₁₋₃ ou une liaison de valence;
Y représente un groupe carboxaldéhyde ou un groupe de formule -C(R⁴)(R⁵)OR¹ où R¹ est tel que défini précédemment et R⁴ et R⁵, qui peuvent être identiques ou différents, sont chacun choisis parmi les atomes d'hydrogène, les groupes alkyle, alcényle et alcynyle, pour que la teneur totale en carbone de R⁴ et R⁵ n'excède pas trois atomes, à la condition que X soit une liaison de valence quand à la fois R⁴ et R⁵ sont différents de l'hydrogène ; et
la ligne en pointillés signifie qu'une double liaison peut être éventuellement présente à la position 16(17).

2. Composés selon la revendication 1, dans lesquels R² représente un groupe hydroxy ou alcoxy en C₁₋₆ et Y est un groupe carboxaldéhyde ou un groupe de formule -C(R⁴)(R⁵)OR¹ où R¹ est tel que défini dans la revendication 1 et R⁴ et R⁵ représentent tous deux des atomes d'hydrogène.

3. Composés selon la revendication 1, dans lesquels Y un groupe de formule -C(R⁴)(R⁵)OR¹ où R¹ est tel que défini dans la revendication 1, l'un de R⁴ et R⁵ est un groupe méthyle, éthyle, vinyle, éthynyle ou propargyle et l'autre est un atome d'hydrogène, ou R⁴ et R⁵ représentent tous deux des groupes méthyle.

4. Composés selon l'une quelconque des revendications précédentes, dans lesquels R¹ est choisi parmi les atomes d'hydrogène, les groupes alkyle en C₁₋₆ et les groupes protecteurs de O labiles dans le métabolisme.

5. Composés selon la revendication 4, dans lesquels lesdits groupes protecteurs de O, labiles dans le métabolisme, sont des groupes sulfamoyle.

6. Composés selon l'une quelconque des revendications précédentes, dans lesquels R² est choisi parmi les groupes méthoxy, éthoxy, propoxy, vinyle, prop-1-ényle, but-1-ényle, hydroxyméthyle, hydroxyéthyle, hydroxypropyle, méthoxyméthyle, méthoxyéthyle, méthoxypropyle, éthoxyméthyle, éthoxyéthyle et éthoxypropyle.

7. Composés selon la revendication 1 :
2-méthoxy-3-hydroxy-19-nor-chol-1,3,5(10),16-tétraèn-24-ol ;
ester 2-méthoxy-3-hydroxy-19-nor-chol-1,3,5(10),16-tétraèn-24-ol-24-sulfamate ;
2-méthoxy-3-hydroxy-20α-hydroxyméthyl-19-nor-prégn-1,3,5(10)-triène ;
2-méthoxy-3-hydroxy-20α-hydroxyméthyl-19-nor-prégn-1,3,5(10),16-tétraène ;
2-méthoxy-3-hydroxy-20α-acétoxyméthyl-19-nor-prégn-1,3,5(10),16-tétraène ;
2-méthoxy-3-hydroxy-20α-acétoxyméthyl-19-nor-prégn-1,3,5(10),16-tétraène-3-O-sulfamate ;
2-méthoxy-3-hydroxy-20α-acétoxyméthyl-19-nor-prégn-1,3,5(10)-triène-3-O-sulfamate ;
2-méthoxy-3-hydroxy-20α-hydroxyméthyl-19-nor-prégn-1,3,5(10),16-tétraène-3-O-sulfamate ;
2-méthoxy-3-hydroxy-20α-hydroxyméthyl-19-nor-prégn-1,3,5(10)-triène-3-O-sulfamate ;
2-éthoxy-3-hydroxy-20α-hydroxyméthyl-19-nor-prégn-1,3,5(10)-triène ;
2-hydroxyméthyl-3-hydroxy-20α-hydroxyméthyl-19-nor-prégn-1,3,5(10)-triène ;
2-propènyl-3-hydroxy-20α-hydroxyméthyl-19-nor-prégn-1,3,5(10)-triène ;
2-méthoxy-3-hydroxy-20α-(1-hydroxyéthyl)-19-nor-prégn-1,3,5(10)-triène ;
2-méthoxy-3-hydroxy-20α-(1-hydroxyprop-2-ynyl)-19-nor-prégn-1,3,5(10)-triène ;
2-méthoxy-3-hydroxy-20α-(1-hydroxybut-3-ynyl)-19-nor-prégn-1,3,5(10)-triène ;
2-méthoxy-3-hydroxy-20α-(2-hydroxyprop-2-yl)-19-nor-prégn-1,3,5(10)-triène ;
2-méthoxy-3-hydroxy-19-nor-prégn-1,3,5(10)-triène-20β-carboxaldéhyde ;
2-méthoxy-3-hydroxy-20β-hydroxyméthyl-19-nor-prégn-1,3,5(10)-triène ;
2-méthoxy-3-hydroxy-20α-(2-oxoéthyl)-19-nor-prégn-1,3,5(10)-triène ;
2-méthoxy-3-hydroxy-20α-(2-hydroxyéthyl)-19-nor-prégn-1,3,5(10)-triène ;
2-méthoxy-3-hydroxy-20α-hydroxyméthyl-19-nor-prégn-1,3,5(10),16-tétraène-3,22-bis-O-sulfamate ; et
2-méthoxy-3-hydroxy-20α-hydroxyméthyl-19-nor-prégn-1,3,5(10),16-tétraène-22-O-sulfamate.

8. Composés actifs de formule (I) selon l'une quelconque des revendications précédentes, destinés à l'utilisation dans la gestion des maladies néoplasiques; à titre d'agents destinés à favoriser la cicatrisation des plaies ; dans le traitement des maladies osseuses, des maladies autoimmunes, de la réaction greffon - hôte, du rejet des transplantations, des maladies inflammatoires, des néoplasies ou hyperplasies, des maladies dermatologiques, de l'hypertension, de l'arthrite rhumatoïde, de l'arthrite psoriatique, de l'asthme, des troubles cognitifs ou de la démence sénile ; dans le contrôle de la fertilité chez les sujets humains ou animaux ; dans la baisse du taux de cholestérol sérique élevé ou dans la gestion des troubles impliquant la coagulation du sang.

9. Composés actifs de formule (I) selon l'une quelconque des revendications 1 à 7, destinés à être utilisés comme antiangiogéniques.

10. Utilisation d'un composé actif de formule (I), selon l'une quelconque des revendications 1 à 7, pour la fabrication d'un médicament destiné à être utilisé dans la gestion des maladies néoplasiques ; à titre d'agents destinés à favoriser la cicatrisation des plaies ; dans le traitement des maladies osseuses, des maladies autoimmunes, de la réaction greffon - hôte, du rejet des transplantations, des maladies inflammatoires, des néoplasies ou hyperplasies, des maladies dermatologiques, de l'hypertension, de l'arthrite rhumatoïde, de l'arthrite psoriatique, de l'asthme, des troubles cognitifs ou de la démence sénile ; dans le contrôle de la fertilité chez les sujets humains ou animaux ; dans la baisse du taux de cholestérol sérique élevé ou dans la gestion des troubles impliquant la coagulation du sang.

11. Compositions pharmaceutiques comprenant un composé actif de formule (I), selon l'une quelconque des revendications 1 à 7, en mélange avec un ou plusieurs supports ou excipients physiologiquement acceptables.
